# EUROPÄISCHE PATENTSCHRIFT

(11) **EP 2 032 198 B1**
(45) Veröffentlichungstag und Bekanntmachung des Hinweises auf die Patenterteilung: **23.01.2013**
(21) Anmeldenummer: 07725093.4
(22) Anmeldetag: 11.05.2007
(51) Int. Cl.: A61M 37/00, A61N 7/00

(54) **ULTRASCHALLVERSTÄRKTES TRANSDERMALES THERAPEUTISCHES SYSTEM**
TRANSDERMAL THERAPEUTIC SYSTEM REINFORCED BY ULTRASOUNDS
SYSTÈME TRANSDERMIQUE THÉRAPEUTIQUE RENFORCÉ PAR DES ULTRASONS

(30) Priorität: 24.06.2006 DE 102006028987
(43) Veröffentlichungstag der Anmeldung: 11.03.2009
(73) Patentinhaber: Horstmann, Michael, Dr., 56564 Neuwied (DE)
(72) Erfinder: HORSTMANN, Michael, 56564 Neuwied (DE); KOCH, Andreas, 56581 Melsbach (DE)
(74) Vertreter: Flaccus, Rolf-Dieter
(86) Internationale Anmeldenummer: PCT/EP2007/004173
(87) Internationale Veröffentlichungsnummer: WO 2007/147461

(56) Entgegenhaltungen:
- WO-A-02/056939
- WO-A-2005/069758
- DE-A1- 19 814 084
- US-A- 4 787 888
- US-A- 5 415 629

## Beschreibung

Die Erfindung betrifft ein transdermales therapeutisches System mit einer pharmazeutischen Wirkstoffzubereitung, mit einer eine Kontaktfläche umfassenden adhäsiven Schicht und mit mindestens einem Ultraschallgeber, wobei der Ultraschallgeber zumindest auf der der Kontaktfläche abgewandten Seite an die pharmazeutische Wirkstoffzubereitung mittelbar oder unmittelbar angrenzt.

Aus der US 4,787,888 ist eine derartige Vorrichtung bekannt. Der Wirkstoff ist in einer hochviskosen Lösung gelöst und in einem Wirkstoffreservoir gelagert, das von einem klebstoffhaltigen Befestigungsflansch umgeben ist. Die hochviskose Lösung dämpft die vom Ultraschallgeber abgegebenen Schwingungen. Die Wirkstoffzufuhr ist auf eine kleine Fläche der Haut beschränkt. Hierdurch ist nur eine langsame Diffusion des Wirkstoffs in die Haut und eine gering Wirkstoffausnutzung möglich. Eine Kombination mit anderen resorptionsfördernden Maßnahen ist nicht möglich.

Auch WO-A-2005-69 758 zeigt eine derartige Vorrichtung.

Der vorliegenden Erfindung liegt daher die Problemstellung zugrunde, ein ultraschallverstärktes transdermales Therapiesystem mit einer hohen Wirkstoffausnutzung zu entwickeln, das eine verbesserte Wirkstoffaufnahme durch die Haut ermöglicht.

Diese Problemstellung wird mit den Merkmalen des Hauptanspruches gelöst. Dazu ist das transdermale therapeutische System ein mehrschichtiges Laminat, wobei eine Laminatschicht den Ultraschallgeber umfasst, eine Laminatschicht die adhäsive Schicht umfasst und eine Laminatschicht die pharmazeutische Wirkstoffzubereitung umfasst. Außerdem sind in einer Draufsicht zumindest bei einer ebenen Lage des transdermalen therapeutischen Systems alle Laminatschichten zumindest annähernd kongruent zueinander und weisen zumindest annähernd die Größe der Kontaktfläche auf.

Weitere Einzelheiten der Erfindung ergeben sich aus den Unteransprüchen und der nachfolgenden Beschreibung schematisch dargestellter Ausführungsformen.
- Figur 1:: Transdermales therapeutisches System bei der Anwendung;
- Figur 2:: Teillängsschnitt durch Figur 1;
- Figur 3:: Therapeutisches System mit Segmentierungen;
- Figur 4:: Therapeutisches System mit einer Membran;
- Figur 5:: Therapeutisches System mit Mikronadeln.

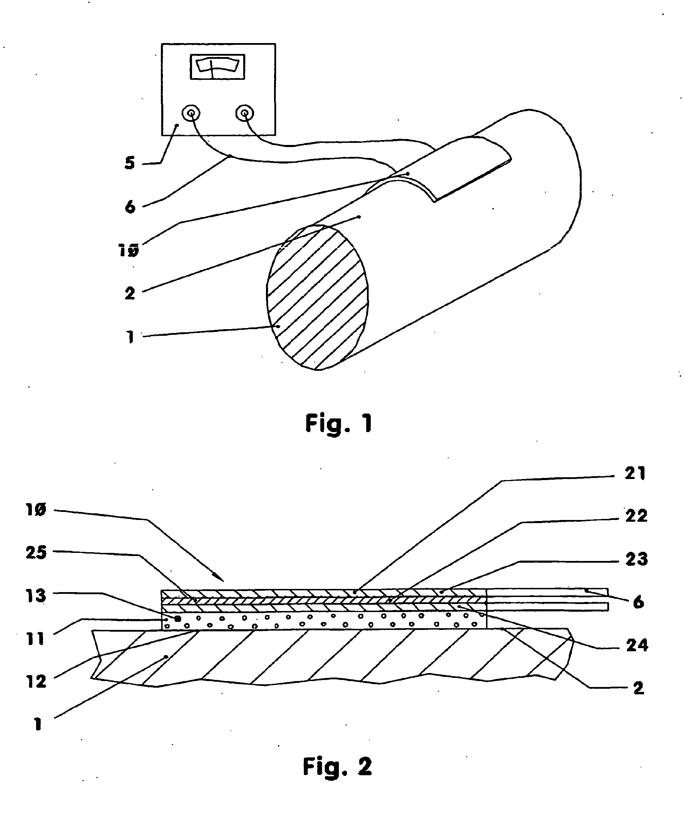

Die Figur 1 zeigt ein transdermales therapeutisches System (10) bei der Anwendung, z.B. an einem Arm (1) eines Patienten. Das transdermale therapeutische System (10) ist eine auf den Arm (1) aufgeklebte, wirkstoffhaltige Arzneiform (10) mit einem integrierten Ultraschallgeber (21). Der Ultraschallgeber (21) ist hier mittels Leitungen (6) an einen Oszillator (5) angeschlossen, der elektrisch mit einer Stromquelle verbunden ist.

Die Figur 2 zeigt einen Teillängsschnitt der Figur 1. Das transdermale therapeutische System (10) ist als mehrschichtiges Laminat aufgebaut. Es umfasst hier als untere Laminatschicht eine mit einer Kontaktfläche (12) auf der Haut (2) des Patienten klebende adhäsive Laminatschicht (11), in die eine pharmazeutische Wirkstoffzubereitung (13) eingelagert ist. Mit dieser ersten Laminatschicht (11) ist vollflächig der Ultraschallgeber (21) als weitere Laminatschicht (21) verbunden. Der Ultraschallgeber (21) ist somit zumindest in einer Draufsicht auf das eben liegende transdermale therapeutische System (10) zumindest annähernd kongruent zur adhäsiven Schicht (11). Beide Schichten (11, 21) haben in diesem Ausführungsbeispiel die Größe der Kontaktfläche (12). Gegebenenfalls liegt zwischen der adhäsiven Schicht (11) und dem Ultraschallgeber (21) eine z.B. inerte Trennfolie, die chemische Reaktionen zwischen der adhäsiven Schicht (11) und dem Ultraschallgeber (21) verhindert.

Das transdermale therapeutische System (10) hat in diesem Ausführungsbeispiel eine quadratische Grundfläche mit einer Kantenlänge von 80 Millimetern. Die Grundfläche kann aber auch rechteckig, rund, etc. sein. Die Dicke des Systems (10) beträgt hier beispielsweise zwischen 500 und 600 Mikrometern.

Die adhäsive Schicht (11) mit der eingelagerten pharmazeutischen Wirkstoffzubereitung (13) ist beispielsweise eine lipophile, halbfeste Klebemasse. In ihr können die einzelnen Bestandteile in separaten Matrizen oder in einer monolithischen Reservoir- und Klebematrix vorliegen. In einer monolithischen Reservoir- und Klebematrix kann die pharmazeutische Wirkstoffzubereitung (13) beispielsweise gelöst oder dispergiert sein.

Die adhäsive Schicht (11) ist hier 200 Mikrometer dick, sie kann aber dünner ausgeführt sein. Die Matrix der adhäsiven Schicht kann Copolymere mit Acrylsäureester, Mischungen aus Kautschuken, Polybutylen, Polyisobutylen und Harze, Polyvinylacetat, Siliconpolymere, etc. umfassen. Diese Werkstoffe sind bei der Anwendung auf der Haut (2) des Patienten unbedenklich. Die Matrix kann bis zu 40 % Füllstoffe enthalten, z.B. Titanoxid, Zinkoxid, Kreide, Aktivkohle, feinverteiltes Siliziumdioxid, etc. Die adhäsive Schicht (11) ist z.B. druckempfindlich. Das bedeutet, dass die Haftwirkung der Schicht (11) auf der Haut (2) unter der Einwirkung eines äußeren Drucks verstärkt wird.

Der Ultraschallgeber (21) ist beispielsweise ein Dickenschwinger mit einem piezoelektrischen Element (22). Das piezoelektrische Element (22) umfasst z.B. eine piezoelektrische Folie (25) und zwei Elektroden (23, 24). Die piezoelektrische Folie (25) besteht z.B. aus einem dielektrischen Werkstoff oder aus einem Werkstoff, der seine piezoelektrischen Eigenschaften durch mechanische oder elektrische Behandlung erhalten hat. Beispielsweise kann durch Anlegen einer Gleichspannung bei einer Temperatur unterhalb der Curietemperatur eine spontane, remanente Polarisation erreicht werden. Die in diesem Ausführungsbeispiel eingesetzte Folie ist eine Polymerfolie, z.B. aus Polyvinylidenfluorid (PVDF), Polyvinylidenchlorid (PVDC), etc. Sie hat beispielsweise eine Dicke zwischen 5 Mikrometern und 25 Mikrometern.

Die Elektroden (23, 24) sind auf beiden Seiten der Folie (25) angeordnet. Beispielsweise ist zur Erzeugung der Elektroden (23, 24) die Folie (25) beidseitig mittels eines Metallbedampfungsprozesses mit einer elektrisch leitfähigen Metallschicht beschichtet. Der Werkstoff der Elektroden (23, 24) ist ein hautverträglicher Werkstoff. Dies kann z.B. Aluminium, Silber, Kupfer, Zink, Gold, ein durch Kohlenstoff leitfähig gemachte Polymerzubereitung, etc. sein. Die Dicke des piezoelektrischen Elements (22) beträgt hier zwischen 10 Mikrometern und 100 Mikrometern, die Dicke kann jedoch bis zu 300 Mikrometern betragen.

Anstatt des hier beschriebenen Dickenschwingers kann der Ultraschallgeber einen Verbundschwinger umfassen, der beispielsweise Zusatzmassen zur Einstellung seiner Resonanzfrequenz hat.

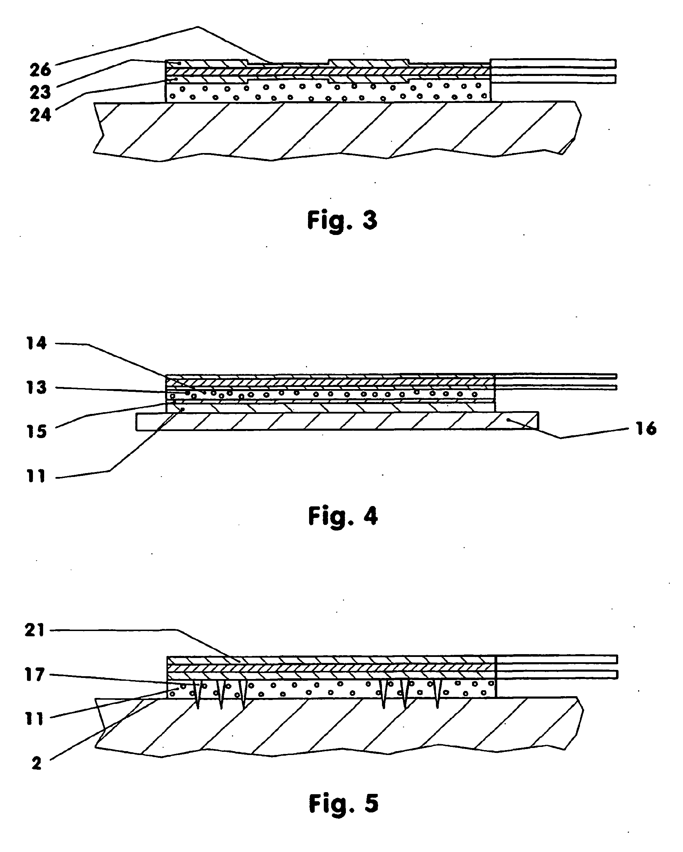

Zur Herstellung des transdermalen therapeutischen Systems (10) wird z.B. die adhäsive Schicht (11) mit der eingelagerten Wirkstoffzubereitung (13) mittels Beschichtungs-, Verdampfungs- und Trocknungsvorgängen hergestellt. Aufgrund der geringen Schichtdicke hat die so hergestellte, beispielsweise gelartige Masse eine weitgehend homogene Zusammensetzung. Beispielsweise wird die adhäsive Schicht (11) auf einer Schutzfolie (16), vgl. Figur 4 aufgetragen. Die Schutzfolie (16) wird erst unmittelbar vor der Anwendung des transdermalen therapeutischen Systems (10) von diesem abgezogen.

Auf die adhäsive Schicht (11) wird, beispielsweise unter Zwischenlage einer Trennfolie, der Ultraschallgeber (21) auflaminiert. Der Ultraschallgeber (21) liegt dann vollflächig auf der adhäsiven Schicht (11) oder auf der Trennfolie auf.

Nach dem Auflaminieren auf die adhäsive Schicht (11) grenzt der Ultraschallgeber (21) zumindest mittelbar an die Wirkstoffzubereitung (13) an. Beispielsweise bei einer hohen Wirkstoffkonzentration kann die untere Elektrode (24) des Ultraschallgebers (21) die Wirkstoffzubereitung (13) unmittelbar kontaktieren.

Um das transdermale therapeutische System (10) einzusetzen, wird dieses nach dem Abziehen der Schutzfolie (16) auf die Haut (2), z.B. des Arms (1) aufgeklebt.

Die Wirkstoffzubereitung (13) dringt durch die adhäsive Schicht (11) und die Haut (2) in den Arm (1). Die Diffusionsrate ist zunächst gering, da die Hornhaut, die äußere Schicht der Haut (2), nur einen geringen Wirkstoffstrom durchlässt.

Der Ultraschallgeber (21) wird elektrisch an den Oszillator (5) angeschlossen und hiernach die Stromquelle eingeschaltet. Die am Ultraschallgeber (21) angelegte Spannung beträgt hier beispielsweise zwischen 10 Volt und 40 Volt. Der Ultraschallgeber (21) kann mit Spannungen zwischen 2 Volt und 1000 Volt betrieben werden. Die Frequenz des angelegten Wechselstroms beträgt in diesem Ausführungsbeispiel zwischen 20 kHz und 100 kHz.

Aufgrund der angelegten elektrischen Spannung zwischen den Elektroden (23, 24) wird die piezoelektrische Folie (25) verformt. Mittels dieses inversen piezoelektrischen Effekts erregt die durch den Oszillator (5) erzeugte Wechselspannung die Folie (25) zu Schwingungen in einer Richtung normal zum Arm (1).

Das schwingende piezoelektrische Element (22) überträgt einen Teil seiner Schwingungsenergie auf die wirkstoffhaltige adhäsive Schicht (11) und in die Haut (2). In der adhäsiven Schicht (11) bewirkt die zugeführte Energie beispielsweise eine Zunahme der kinetischen Energie der pharmazeutischen Wirkstoffzubereitung (13). Die in die Haut (2) übertragenen Schwingungen erfahren z.B. aufgrund der gelartigen, dünnen adhäsiven Schicht (11) auf ihrem Weg nur eine geringe Dämpfung. Durch die Zufuhr der Ultraschallenergie wird die Hornhaut aktiviert und wirkstoffdurchlässig. Die Sperrwirkung der Hornhaut wird wirksam durchbrochen. Die Diffusion der Wirkstoffzubereitung (13) durch die adhäsive Schicht (11) und die Haut (2) wird verstärkt. Die Diffusion in die Haut (2) erfolgt hierbei über die gesamte Kontaktflache (12) des transdermalen therapeutischen Systems (10) mit der Haut (2). Außerdem ermöglicht die große Kontaktfläche (12) eine weitgehend schmerzfreie Übertragung der Wirkstoffe durch die Haut (2). Aufgrund der geringen Schichtdicke der wirkstoffhaltigen adhäsiven Schicht (11) sind die Diffusionswege kurz. Hierdurch wird eine hohe Wirkstoffausnutzung erreicht.

In der Figur 3 ist ein weiteres transdermales therapeutisches System (10) dargestellt. In diesem System (10) weisen die Elektroden (23, 24) Segmentierungen (26), z.B. Durchbrüche auf. Beim Betrieb des transdermalen therapeutischen Systems (10) werden die Bereiche der Segmentierungen (26) nur geringfügig erregt. So kann beispielsweise die Wirkstoffabgabe durch die Haut (2) in einigen Bereichen begrenzt werden, während in anderen Bereichen eine durch den Ultraschallgeber (21) verstärkte Wirkstoffabgabe bewirkt wird.

In der Figur 4 ist ein transdermales therapeutisches System (10) dargestellt, in dem die Wirkstoffzubereitung (13) in einer wirkstoffhaltigen Schicht (14) eingelagert ist. Diese Schicht (14) hat beispielsweise eine gelartige Konsistenz. Sie ist z.B. 300 Mikrometer dick und ist hier durch eine zumindest bereichsweise für die Wirkstoffzubereitung (13) durchlässige Membran (15) von der adhäsiven Schicht (11) getrennt. Die Dicke der Membran beträgt zwischen 10 Mikrometer und 50 Mikrometer. Auf der der Kontaktfläche (12) abgewandten Seite grenzt der Ultraschallgeber (21) an die wirkstoffhaltige Schicht (14) an. Der Ultraschallgeber (21) ist beispielsweise so aufgebaut, wie im Zusammenhang mit der Figur 2 oder der Figur 3 beschrieben. Gegebenenfalls kann zwischen dem Ultraschallgeber (21) und der wirkstoffhaltigen Laminatschicht (14) eine Trennfolie angeordnet sein. Das transdermale therapeutische System ist hier - vor der Anwendung - mittels einer Schutzfolie (16) geschützt.

Bei der Anwendung des transdermalen therapeutischen Systems (10) durchdringt die Wirkstoffzubereitung (13) die beispielsweise semipermeable Membran (15) und diffundiert durch die adhäsive Schicht (11) und durch die Haut (2). Unter dem Einfluss des Ultraschallgebers (21) wird die Diffusion verstärkt, wie oben beschrieben. Die große Kontaktfläche (12) ermöglicht auch in diesem Ausführungsbeispiel eine weitgehend schmerzfreie Anwendung und eine gute Wirkstoffausnutzung.

In allen beschriebenen Ausführungsbeispielen kann das piezoelektrische Element (22) separate Elektroden (23, 24) aufweisen, die an der piezoelektrischen Folie (25) anliegen. Auch kann die kontaktflächenferne Elektrode (23) starr ausgebildet und die andere Elektrode (24) schwingfähig sein. Das gesamte transdermale therapeutische System (10) kann mittels eines Bandes, z.B. eines Textilbandes, geschützt sein. Dieses Band ist dann so angeordnet, dass es die Schwingungen des piezoelektrischen Elements (22) nicht behindert.

Der Oszillator (5) kann am transdermalen therapeutischen System (10) angeordnet sein. Bei der Anwendung eines derartigen Systems (10) wird der Oszillator (5) an eine Gleich- oder Wechselstromquelle angeschlossen.

Es ist auch denkbar, den Ultraschallgeber (21) mit kapazitiv wirkenden Elementen auszuführen.

In der Figur 5 ist ein ultraschallverstärktes transdermales therapeutisches System (10) dargestellt, das eine zusätzliche Resorptionsverstärkung aufweist. Um die Wirkstoffzufuhr durch die Haut (2) zu verbessern, umfasst das transdermale therapeutische System (10) beispielsweise in Gruppen angeordnete Mikronadeln (17). Diese Mikronadeln (17) sind z.B. an der unteren Elektrode (24) des Ultraschallgebers (21) befestigt und ragen zumindest bei der Anwendung des transdermalen therapeutischen Systems (10) um wenige Zehntel Millimeter aus der Kontaktfläche (12) heraus. Hierbei kann das transdermale Therapeutische System (10) so aufgebaut sein, wie im Zusammenhang mit den vorgenannten Figuren 2 - 4 beschrieben.

Bei der Anwendung des in der Figur 5 dargestellten transdermalen therapeutischen Systems (10) durchdringen die Mikronadeln die Hornhaut. Hierbei fixieren sie das transdermale therapeutische System (10) und spannen beispielsweise die Hornhaut zwischen den Nadeln (17), wodurch die Hornhaut bereichsweise oder punktuell wirkstoffdurchlässig wird. Der schwingende Ultraschallgeber (21) bewirkt einen Arbeitshub der Mikronadeln (17). Hierdurch wird die vom Ultraschallgeber (21) abgegebene Energie zusätzlich punktförmig auf die Haut (2) übertragen. Dies bewirkt eine verbesserte Wirkstoffdiffusion durch die Haut (2).

Die Mikronadeln (17) weisen beispielsweise Hinterschneidungen auf. Diese wirken beim Einsatz des transdermalen therapeutischen Systems (10) als Widerhaken. Es ist auch denkbar, die Mikronadeln (17) mit einer schraubenförmigen Kontur auszubilden. Diese Mikronadeln (17) sind dann beispielsweise in axialer und in radialer Richtung an der unteren Elektrode (24) gelagert. Sie sind somit frei drehbar gelagert. Die schwingende Elektrode (24) des Ultraschallgebers (21) bewirkt dann eine Rotation der Mikronadel (17) in der Art einer Bohrbewegung beim Eindringen in die Hornhaut. Bei diesen transdermalen therapeutischen Systemen (10) können die Mikronadeln,(17) auch so angeordnet sein, dass sie vor dem Einsatz des Systems (10) nicht aus der Kontaktfläche (12) herausragen.

Auch der Einsatz chemischer Permeationsverstärker in der adhäsiven Schicht (11) ist denkbar. Dies können flüssige Zusatzstoffe wie z.B. Alkohole, Fettsäuren, Carbonsäuren, Ester etc. sein. Sie können auf der gesamten Kontaktfläche (12) auf die Haut (2) einwirken. Die Permeation des Wirkstoffs durch die Haut (2) wird hierdurch zusätzlich verstärkt.

### Bezugszeichenliste:

- 1: Arm
- 2: Haut

- 5: Oszillator
- 6: Leitungen

- 10: Transdermales therapeutisches System, wirkstoffhaltige Arzneiform
- 11: adhäsive Schicht, Laminatschicht
- 12: Kontaktfläche
- 13: pharmazeutische Wirkstoffzubereitung
- 14: wirkstoffhaltige Schicht, Laminatschicht
- 15: Membran
- 16: Schutzfolie
- 17: Mikronadeln

- 21: Ultraschallgeber, Laminatschicht
- 22: piezoelektrisches Element
- 23: Elektroden, fern von (3)
- 24: Elektrode, an (13) mittelbar oder unmittelbar angrenzend
- 25: Folie
- 26: Segmentierungen

## Patentansprüche

1. Transdermales therapeutisches System (10) mit einer pharmazeutischen Wirkstoffzubereitung (13), mit einer adhäsiven Schicht (11), die eine Kontaktfläche (12) für den Kontakt mit der Haut aufweist, und mit mindestens einem Ultraschallgeber (21), wobei der Ultraschallgeber (21) zumindest auf der der Kontaktfläche (12) abgewandten Seite an die pharmazeutische Wirkstoffzubereitung (13) mittelbar oder unmittelbar angrenzt,
- wobei das transdermale therapeutische System (10) ein mehrschichtiges Laminat ist, das einen Ultraschallgeber (21) als eine Laminatschicht und die adhäsive Schicht (11) als eine weitere Laminatschicht umfasst,
- wobei die pharmazeutische Wirkstoffzubereitung (13) in der adhäsiven Schicht (11) oder in einer wirkstoffhaltigen Laminatschicht (14) eingelagert ist,
und
- wobei in in einer Draufsicht zumindest bei einer ebenen Lage des transdermalen therapeutischen Systems (10) alle Laminatschichten (11, 21; 11, 14, 21) zumindest annähernd kongruent zueinander sind und zumindest annähernd die Größe der Kontaktfläche (12) der adhäsiven Schicht (11) aufweisen.

2. Transdermales therapeutisches System nach Anspruch 1, **dadurch gekennzeichnet, dass** die Dicke einer einzelnen Schicht (11, 14) kleiner ist als 300 Mikrometer.

3. Transdermales therapeutisches System nach Anspruch 1, **dadurch gekennzeichnet, dass** die Gesamtdicke des transdermalen therapeutischen Systems (10) kleiner ist als 600 Mikrometer.

4. Transdermales therapeutisches System nach Anspruch 1, **dadurch gekennzeichnet, dass** zwischen der adhäsiven Schicht (11) und einer Schicht (14), die die Wirkstoffzubereitung (13) umfasst, eine zumindest wirkstoffdurchlässige Membran (15) angeordnet ist.

5. Transdermales therapeutisches System nach Anspruch 1, **dadurch gekennzeichnet, dass** die adhäsive Schicht (11) eine lipophile, druckempfindliche Masse umfasst.

6. Transdermales therapeutisches System nach Anspruch 1, **dadurch gekennzeichnet, dass** der Ultraschallgeber (21) ein piezoelektrisches Element (22) umfasst.

7. Transdermales therapeutisches System nach Anspruch 6, **dadurch gekennzeichnet, dass** das piezoelektrische Element (22) eine piezoelektrische Polymerfolie (25) umfasst

8. Transdermales therapeutisches System nach Anspruch 1, **dadurch gekennzeichnet, dass** es Mikronadeln (17) umfasst, die am Ultraschallgeber (21) befestigt sind oder an diesem gelagert sind.

9. Transdermales therapeutisches System nach Anspruch 8, **dadurch gekennzeichnet, dass** die Mikronadeln (17) zumindest bei der Anwendung des transdermalen therapeutischen Systems (10) aus der Kontaktfläche (12) herausragen.

10. Transdermales therapeutisches System nach Anspruch 8, **dadurch gekennzeichnet, dass** die Mikronadeln (17) Widerhaken aufweisen.

11. Transdermales therapeutisches System nach Anspruch 8, **dadurch gekennzeichnet, dass** die Mikronadeln (17) eine schraubenförmige Kontur aufweisen und in axialer und in radialer Richtung am Ultraschallgeber (21) gelagert sind.

12. Transdermales therapeutisches System nach Anspruch 1, **dadurch gekennzeichnet, dass** die adhäsive Schicht (11) chemische Permeatoren umfasst.

## Claims

1. Transdermal therapeutic system (10) with a pharmaceutical active substance formulation (13) which comprises an adhesive layer (11) comprising a contact surface (12) for contact with the skin, and with at least one ultrasound transmitter (21), said ultrasound transmitter (21), at least on the side directed away from the contact surface (12), being directly or indirectly adjacent to the pharmaceutical active substance formulation (13),
- the transdermal therapeutic system (10) being a multi-layered laminate comprising an ultrasound transmitter (21) as one laminate layer and comprising the adhesive layer (11) as a further laminate layer,
- the pharmaceutical active substance formulation (13) being incorporated in the adhesive layer (11) or in an active substance-containing laminate layer (14),
and
- in a top view, at least with the transdermal therapeutic system (10) lying flat, all of the laminate layers (11, 21; 11, 14, 21) being at least approximately congruent to one another and being at least approximately the size of the contact surface (12) of the adhesive layer (11).

2. Transdermal therapeutic system according to Claim 1, **characterized in that** the thickness of an individual layer (11, 14) is less than 300 micrometers.

3. Transdermal therapeutic system according to Claim 1, **characterized in that** the total thickness of the transdermal therapeutic system (10) is less than 600 micrometers.

4. Transdermal therapeutic system according to Claim 1, **characterized in that** a membrane (15), at least permeable to active substance, is arranged between the adhesive layer (11) and a layer (14) comprising the active substance formulation (13).

5. Transdermal therapeutic system according to Claim 1, **characterized in that** the adhesive layer (11) comprises a lipophilic, pressure-sensitive composition.

6. Transdermal therapeutic system according to Claim 1, **characterized in that** the ultrasound transmitter (21) comprises a piezoelectric element (22).

7. Transdermal therapeutic system according to Claim 6, **characterized in that** the piezoelectric element (22) comprises a piezoelectric polymer film (25).

8. Transdermal therapeutic system according to Claim 1, **characterized in that** it comprises microneedles (17) which are attached to the ultrasound transmitter (21), or are mounted thereon.

9. Transdermal therapeutic system according to Claim 8, **characterized in that** the microneedles (17) protrude from the contact surface (12), at least during use of the transdermal therapeutic system (10).

10. Transdermal therapeutic system according to Claim 8, **characterized in that** the microneedles (17) have barbs.

11. Transdermal therapeutic system according to Claim 8, **characterized in that** the microneedles (17) have a screw-shaped contour and are mounted in the axial and radial directions on the ultrasound transmitter (21).

12. Transdermal therapeutic system according to Claim 1, **characterized in that** the adhesive layer (11) comprises chemical permeation enhancers.

## Revendications

1. Système thérapeutique transdermique (10) présentant une préparation pharmaceutique (13) de substance active,
le système comprenant une couche adhésive (11) qui présente une surface de contact (12) pour le contact avec la peau et
au moins un émetteur (21) d'ultrasons; l'émetteur (21) d'ultrasons étant directement ou indirectement adjacent à la préparation pharmaceutique (13) de substance active au moins sur son côté non tourné vers la surface de contact (12),
- le système thérapeutique transdermique (10) étant un stratifié multicouche, comprenant un émetteur (21) d'ultrasons comme une couche du stratifié et la couche adhésive (11) comme autre couche de stratifié
- la préparation pharmaceutique (13) de substance active étant incorporée dans la couche adhésive (11) ou dans une couche du stratifié comprenant de la substance active, et
- au moins lorsque le système thérapeutique transdermique (10) est posé à plat, toutes les couches (11, 21; 11, 14, 21) du stratifié vues en plan étant au moins approximativement congruentes les unes par rapport aux autres et présentant au moins approximativement la taille de la surface de contact (12) de la couche adhésive (11).

2. Système thérapeutique transdermique selon la revendication 1, **caractérisé en ce que** l'épaisseur d'une couche (11, 14) est inférieure à 300 micromètres.

3. Système thérapeutique transdermique selon la revendication 1, **caractérisé en ce que** l'épaisseur totale du système thérapeutique transdermique (10) est inférieure à 600 micromètres.

4. Système thérapeutique transdermique selon la revendication 1, **caractérisé en ce qu'**une membrane (15) perméable au moins vis-à-vis de la substance active est disposée entre la couche adhésive (11) et une couche (14) qui contient la préparation (13) de substance active.

5. Système thérapeutique transdermique selon la revendication 1, **caractérisé en ce que** la couche adhésive (11) comporte une pâte lipophile et sensible à la pression.

6. Système thérapeutique transdermique selon la revendication 1, **caractérisé en ce que** l'émetteur (21) d'ultrasons comprend un élément piézoélectrique (22).

7. Système thérapeutique transdermique selon la revendication 6, **caractérisé en ce que** l'élément piézoélectrique (22) comporte une feuille (25) en polymère piézoélectrique.

8. Système thérapeutique transdermique selon la revendication 1, **caractérisé en ce qu'**il comprend des micro-aiguilles (17), qui sont fixées à l'émetteur d'ultrasons (21) ou montées sur celui-ci.

9. Système thérapeutique transdermique selon la revendication 8, **caractérisé en ce que** les micro-aiguilles (17) débordent hors de la surface de contact (12) au moins lorsque le système thérapeutique transdermique (10) est utilisé.

10. Système thérapeutique transdermique selon la revendication 8, **caractérisé en ce que** les micro-aiguilles (17) présentent des contre-crochets.

11. Système thérapeutique transdermique selon la revendication 8, **caractérisé en ce que** les micro-aiguilles (17) présentent un contour hélicoïdal et sont montées dans la direction axiale et la direction radiale sur l'émetteur d'ultrasons (21).

12. Système thérapeutique transdermique selon la revendication 1, **caractérisé en ce que** la couche adhésive (11) comporte des agents chimiques de perméation.
